# EUROPEAN PATENT APPLICATION

(11) **EP 4 603 400 A1**
(43) Date of publication of application: **20.08.2025**
(21) Application number: 25155136.2
(22) Date of filing: 31.01.2025
(51) Int. Cl.: B65B 55/10, C02F 1/72, A61L 2/18

(54) **APPARATUS FOR DISPOSING OF AN EXHAUSTED HYDROGEN PEROXIDE SOLUTION, PACKAGING MACHINE PROVIDED WITH SAID APPARATUS, METHOD FOR DISPOSING OF AN EXHAUSTED HYDROGEN PEROXIDE SOLUTION AND METHOD FOR PRODUCING SEALED PACKAGES**

(30) Priority: 13.02.2024 IT 202400003013
(71) Applicant: Tetra Laval Holdings & Finance S.A., 1009 Pully (CH)
(72) Inventor: CARRAROLI, Piero Luigi, 41123 Modena (IT); MONZANI, Federico, 41123 Modena (IT); DONATI, Andrea, 41123 Modena (IT); HULTEBERG, Christian, 22186 Lund (SE); SAEIDIHAGHI, Arash, 22186 Lund (SE); TUNÅ, Per, 22186 Lund (SE); SELIMI, Jon, 22186 Lund (SE); SCHMIDT, Manfred, 22186 Lund (SE)
(74) Representative: Tetra Pak Patent Attorneys

(57) **Abstract**

There is described an apparatus (17) for disposing of an exhausted hydrogen peroxide solution used for the sterilization of a web (3) of packaging material, the apparatus (17) with a catalytic reactor (24) configured for the catalytic decomposition of the exhausted hydrogen peroxide solution, wherein the catalytic reactor (24) comprises a housing (25) containing at least one catalyst bed and at least a supply duct (29) supplying the exhausted hydrogen peroxide solution into the housing (25), a first pipe (37) connected to the housing (25), and a second pipe (38) connected to the housing (25). The catalytic reactor (24) is configured to produce water in liquid phase drained out through the first pipe (37) and a gaseous flow of oxygen and water drained out through the second pipe (38).

## Description

### TECHNICAL FIELD

The present invention relates to apparatus for disposing of an exhausted hydrogen peroxide solution and to a packaging machine provided with an apparatus for disposing of an exhausted hydrogen peroxide solution. The present invention further relates to a method for disposing of an exhausted hydrogen peroxide solution used for the sterilization of a web of packaging material and to a method for producing sealed packages containing a pourable product from a web of packaging material.

### BACKGROUND ART

As is known, many liquid or pourable food products, such as fruit juice, UHT (ultra-high-temperature treated) milk, wine, tomato sauce, etc., are sold in packages made of sterilized packaging material.

A typical example is a parallelepiped-shaped package for liquid or pourable food products, which is made by sealing and folding laminated strip packaging material. The packaging material has a multilayer structure comprising a base layer, e.g. of paper, covered on both sides with layers of heat-seal plastic material, e.g. polyethylene. In the case of aseptic packages for long-storage products, such as UHT milk, the packaging material also comprises a layer of oxygen-barrier material (an oxygen-barrier layer), e.g. an aluminum foil, which is superimposed on a layer of heat-seal plastic material and is in turn covered with another layer of heat-seal plastic material forming the inner face of the package eventually contacting the food product.

Packages of this sort are normally produced on fully automatic packaging machines, which advance a web of packaging material through a sterilization apparatus for sterilizing the web of packaging material at a sterilization station and to an isolation chamber (a closed and sterile environment) in which the sterilized web of packaging material is maintained and advanced. During advancement of the web of packaging material through the isolation chamber, the web of packaging material is folded and sealed longitudinally at a tube forming station to form a tube having a longitudinal seam portion, the tube being further fed along a vertical advancing direction.

For completing the forming operations, the tube is filled with a pourable product, in particular a pourable food product, and is transversally sealed and subsequently cut along equally spaced transversal cross sections within a package forming apparatus of the packaging machine during advancement along the vertical advancing direction.

Pillow packages are so obtained, each pillow package has a longitudinal sealing band, a top transversal sealing band and a bottom transversal sealing band.

Especially for food product it is required to sterilize the web of packaging material prior to filling. Thus, a typical packaging machine comprises a conveying device for advancing the web of packaging material, the sterilization apparatus for sterilizing the web of packaging material prior to its formation into the tube, a tube forming and sealing device configured to form the tube from the advancing web of packaging material and to longitudinally seal the tube, a filling device for filling the tube with the pourable product and the package forming apparatus adapted to form, transversally seal and cut individual packages from the tube of packaging material.

The sterilization apparatus has a sterilizing bath, in which a chemical sterilizing agent is applied to the web of packaging material. Advantageously, the chemical sterilizing agent comprises a hydrogen peroxide solution.

An example of a machine comprising a sterilization apparatus with a sterilizing bath is disclosed in patent document EP2296978B1, which also describes a device, comprising a catalyst, for measuring a concentration of hydrogen peroxide.

The hydrogen peroxide solution used in the sterilization apparatus must be replaced periodically; therefore, the exhausted hydrogen peroxide solution must be disposed at pre-established time interval (for example, every week) and in a safe manner. The packaging machine is therefore provided with an apparatus for disposing of the exhausted hydrogen peroxide solution.

Even though the known apparatuses for disposing of the exhausted hydrogen peroxide solution provide for good results and works satisfactorily well, a need is felt within the industry to further improve the operation, in particular to reduce the consumed amount of water.

### DISCLOSURE OF INVENTION

It is therefore an object of the present invention to provide an apparatus for disposing of an exhausted hydrogen peroxide solution addressing the drawbacks of the prior art and, in particular, being easy and economical to be manufactured.

A further object of the present invention is to provide a packaging machine provided with an apparatus for disposing of an exhausted hydrogen peroxide solution addressing the drawbacks of the prior art and, in particular, being easy and economical to be manufactured.

A further object of the present invention is to provide a method for disposing of an exhausted hydrogen peroxide solution used for the sterilization of a web of packaging material addressing the drawbacks of the prior art and, in particular, being easy and economical to be implemented.

A further object of the present invention is to provide a method for producing sealed packages containing a pourable product from a web of packaging material addressing the drawbacks of the prior art and, in particular, being easy and economical to be implemented.

According to the invention there are provided an apparatus for disposing of an exhausted hydrogen peroxide solution, a packaging machine provided with an apparatus for disposing of an exhausted hydrogen peroxide solution, a method for disposing of an exhausted hydrogen peroxide solution used for the sterilization of a web of packaging material and a method for producing sealed packages containing a pourable product from a web of packaging material according to the appended claims.

### BRIEF DESCRIPTION OF THE DRAWINGS

A non-limiting embodiment of the present invention will be described by way of example with reference to the accompanying drawings, in which:
- figure 1 is a schematic view of a packaging machine having an apparatus for disposing of an exhausted hydrogen peroxide solution according to the present invention, with parts removed for clarity;
- figure 2 is a schematic side view of the packaging machine of figure 1;

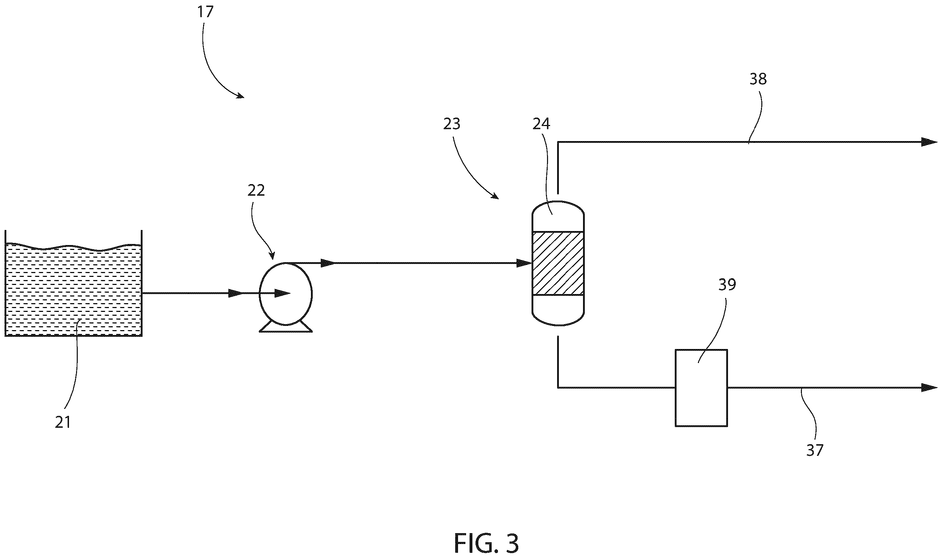

- figure 3 is a schematic view of the apparatus for disposing of an exhausted hydrogen peroxide solution according to the present invention, with parts removed for clarity;

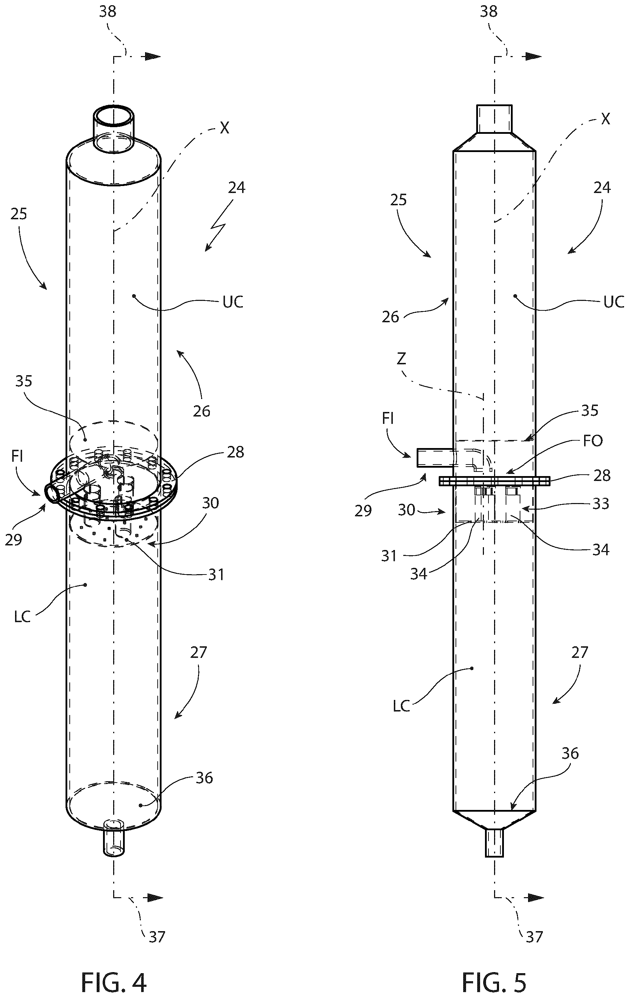

- figure 4 is a perspective view of a catalytic reactor of the apparatus of figure 3;
- figure 5 is a sectional view of the catalytic reactor of figure 4;

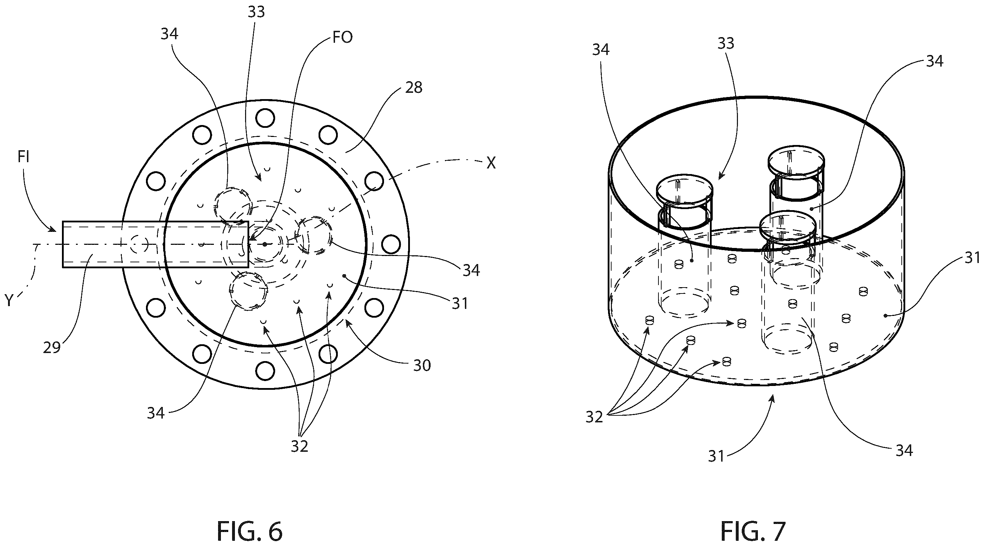

- figure 6 is a plan sectional view of the catalytic reactor of figure 4; and
- figure 7 is a perspective view of a particular of the catalytic reactor of figure 4, with parts removed for clarity.

### BEST MODES FOR CARRYING OUT THE INVENTION

Number 1 indicates as a whole a packaging machine for producing sealed packages 2 of a pourable product, in particular a pourable food product, such as milk, milk drinks, yoghurt, yoghurt drinks, fruit juice, wine, tomato sauce, emulsions, beverages containing pulp, salt, sugar, etc. In more detail, packaging machine 1 may be configured to produce packages 2 from a multilayer packaging material, preferentially, a multilayer packaging material having heat seal properties (i.e. portions of the multilayer packaging material can be sealed to one another).

In further detail, the multilayer packaging material may comprise at least one layer of fibrous material, such as paper or cardboard, and at least two layers of heat-seal plastic material, e.g. polyethylene, interposing the layer of fibrous material in between one another. Preferentially, one of these two layers of heat-seal plastic material may define the inner face of packages 2 contacting the pourable product. Moreover, the multilayer packaging material may also comprise a layer of gas- and light-barrier material, e.g. aluminum foil or ethylene vinyl alcohol (EVOH) film, preferentially being arranged between one of the layers of the heat-seal plastic material and the layer of fibrous material. Preferentially, the packaging material may also comprise a further layer of heat-seal plastic material being interposed between the layer of gas- and light-barrier material and the layer of fibrous material.

In further detail, the multilayer packaging material may be provided in the form of a web 3.

Furthermore, packaging machine 1 is configured to produce packages 2 by forming a tube 4 from the web 3, longitudinally sealing the tube 4, filling the tube 4 with the pourable product and to transversally seal, and preferentially transversally cut the tube 4.

According to some possible non-limiting embodiments, each package 2 extends along a longitudinal axis and comprises at least a first transversal sealing band 5, and preferentially also a second transversal sealing band arranged at opposite ends of the package 2.

Moreover, each package 2 also comprises a longitudinal seam portion 6. Preferentially, each first transversal sealing band 5 and/or each second transversal sealing band are transversal (preferentially perpendicular) to the respective longitudinal seam portion 6.

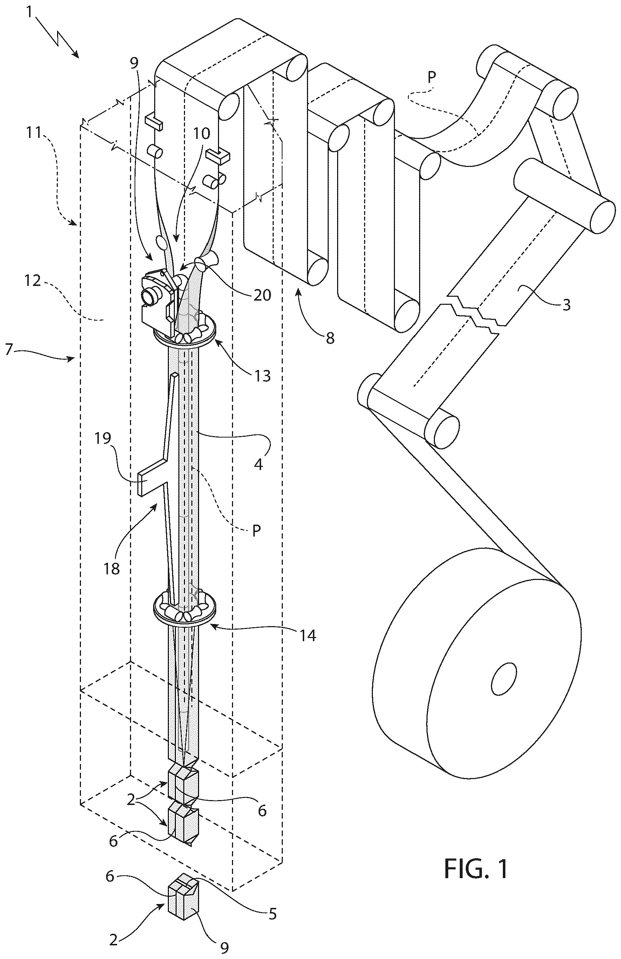

With particular reference to figure 1, packaging machine 1 comprises a package forming apparatus 7 configured to at least partially form the tube 4, preferentially to also transversally seal and/or to transversally cut the tube 4, for obtaining the packages 2.

Moreover, the packaging machine 1 comprises:
- a conveying device 8 configured to advance along a conveying path P the web 3 and the tube 4;
- a tube forming and sealing device 9 configured to form the tube 4 from the advancing web 3 and to longitudinally seal the tube 4; and
- a filling device 10 for filling the tube 4 with the pourable product.

In further detail, the packaging machine 1 comprises an isolation chamber 11, preferentially delimiting an inner environment 12 from an outer environment. Preferentially, the inner environment 12 is a sterile (aseptic) environment, preferably containing a controlled atmosphere. Preferentially, the tube forming and sealing device 9 is at least partially arranged within the isolation chamber 11, in particular within the inner environment 12, and is configured to fold and longitudinally seal the tube 4 within the isolation chamber 11, in particular within the inner environment 12.

The tube forming and sealing device 9 comprises at least two forming assemblies 13, 14 preferentially arranged within the isolation chamber 11, even more preferentially arranged within the inner environment 12, configured to gradually fold in cooperation with one another the web 3 of packaging material into the tube 4, preferentially by overlapping opposite longitudinal edges of the web 3 of packaging material with one another. Thereby, in use, the seam portion 6 is formed. The forming assemblies 13, 14 are spaced apart from, and parallel to, one another. Furthermore, the forming assemblies 13, 14 are arranged coaxial to one another. In particular, the tube forming and sealing device 9 comprises the forming assembly 13 and the forming assembly 14 placed along the conveying path P in succession (one after the other) carried by a fixed structure and interacting with the web 3 of packaging material.

Additionally, the tube forming and sealing device 9 comprises a sealing device 18 having a sealing head 19, preferentially arranged within the isolation chamber 11, even more preferentially within the inner environment 12, and configured to longitudinally seal the tube 4, preferentially along the longitudinal seam portion 6.

More specifically, the sealing head 19 is configured to transfer thermal energy to the tube 4, in particular to the longitudinal seam portion 6 for longitudinally sealing the tube 4. The sealing head 19 can be of the kind operating by means of induction heating and/or by a stream of a heated gas and/or by means of ultrasound and/or by laser heating and/or by any other means.

Preferentially, the filling device 10 comprises a filling pipe 20 configured to direct the pourable product into the tube 4. Preferentially, the filling pipe 20 is at least partially placed within the tube 4 for continuously feeding, in use, the pourable product into the tube 4.

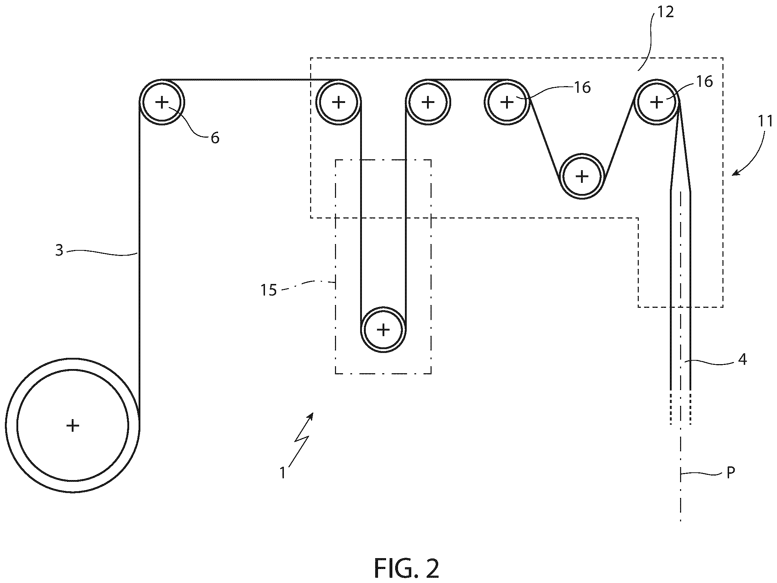

Moreover, as shown in figure 2, the packaging machine 1 comprises a sterilization unit 15 configured to sterilize the advancing web 3 of packaging material. Preferentially the sterilization unit 15 is arranged upstream of the tube forming and sealing device 9 along the conveying path P. The web 3 of packaging material is fed along the conveying path P by means of guiding elements 16, in particular by a number of rollers 16. The sterilization unit 15 has a sterilizing bath, in which a chemical sterilizing agent is applied to the web 3 of packaging material. Advantageously, the chemical sterilizing agent comprises a hydrogen peroxide solution.

In particular, in the hydrogen peroxide solution the hydrogen peroxide is diluted with water. Preferably, the concentration of the hydrogen peroxide solution is 35%. In other words, the hydrogen peroxide solution comprises 35% mass of hydrogen peroxide and 65% mass of water.

The hydrogen peroxide solution used in the sterilization unit 15 must be replaced periodically; therefore, the exhausted hydrogen peroxide solution must be disposed at pre-established time interval (for example, every week) and in a safe manner. Furthermore, the hydrogen peroxide solution must be disposed when a concentration of hydrogen peroxide increases or decreases beyond predetermined values. The packaging machine 1 comprises an apparatus 17 for disposing of the exhausted hydrogen peroxide solution.

The apparatus 17 for disposing of the exhausted hydrogen peroxide solution comprises a disposal tank 21, configured to collect the exhausted hydrogen peroxide solution drained from the sterilizing unit 15.

The apparatus 17 for disposing of the exhausted hydrogen peroxide solution further comprises feeding means 22 which are configured to draw the exhausted hydrogen peroxide solution out the disposal tank 21 and to feed the exhausted hydrogen peroxide solution to a device 23 for the catalytic decomposition and the separation of the exhausted hydrogen peroxide solution. According to a preferred embodiment, the feeding means 22 comprise pumping means 22.

The exhausted hydrogen peroxide solution is fed from the feeding means 22 to the device 23 for the catalytic decomposition and the separation of the exhausted hydrogen peroxide solution in a liquid phase and a gaseous (or aerosol) phase. The flow rate of exhausted hydrogen peroxide solution fed from the feeding means 22 to the device 23 for the catalytic decomposition and the separation of the exhausted hydrogen peroxide solution is comprised between 0.08 l/min and 0.2 l/min. The applicant has noted indeed that flow rates higher than 1 l/min result in the exothermic reaction in the catalyzer generating too much heat, that is then difficult to dissipate.

The temperature of the exhausted hydrogen peroxide solution fed from the feeding means 22 to the device 23 for the catalytic decomposition and the separation of the exhausted hydrogen peroxide solution is comprised between 85°C and 90°C; preferably, the temperature of the exhausted hydrogen peroxide solution is 87°C.

According to some embodiments, the apparatus 17 further comprises a filter, in particular a chemical filter. The filter is positioned upstream of supplying duct. In particular, the filter is interposed between feeding means 22 and device 23. The filter is configured to remove from the hydrogen peroxide solution a stabilizer content (such as phytic acid or phosphoric acid). Hence, the hydrogen peroxide solution is purified from the stabilizer content before being supplied into the housing 25. In such a way, it has been observed that the lifetime of device 23 (in particular catalyst beds) may be increased.

The apparatus 17 (in particular, the device 23 for the catalytic decomposition and the separation of the exhausted hydrogen peroxide solution) comprises a catalytic reactor 24. The catalytic reactor is configured to allow decomposition of hydrogen peroxide into water and oxygen (2 H2O2 --> 2 H2O+ O2). The catalytic reactor 24 comprises a housing 25.

The housing 25 extends along a vertical direction, in particular parallel to the weight force. In the example shown, the housing 25 has a tubular shape having a cylindrical symmetry about an axis X (parallel to the vertical direction). In other examples, the housing 25 may have other shapes e.g. parallelepiped.

The housing 25 is preferably realized in a metal material, in particular stainless steel. The catalytic reactor 24 comprises an upper (tubular) portion 26 and a lower (tubular) portion 27 connected to each other, in a non-releasably manner or in a releasably manner. In particular, the upper portion 26 and the lower portion 27 are hold together by a flange 28. Preferably, the upper portion 26 has a greater height along axis X than the lower portion 25.

The catalytic reactor 24 comprises at least a supply duct 29 having an axis Y. The axis Y is transversal to the axis X; preferably, the axis Y is orthogonal to the axis X. The supply duct 29 is provided with a fluid inlet FI which is in fluid communication with the feeding means 22, from which it receives the exhausted hydrogen peroxide solution. The supply duct 29 is further provided with a fluid outlet FO which is configured to direct the exhausted hydrogen peroxide solution fed from the feeding means 22 into the housing 25. The supply duct 29 is preferably realized in a metal material, in particular stainless steel.

The supply duct 29 is arranged substantially at a medium height of the housing 25, preferably in the lower portion 27. Advantageously, the supply duct 29 has a diameter comprised between 0,7 cm and 3,8 cm; preferably, the supply duct 29 has a diameter of 1,2 cm.

According to a preferred embodiment, the supply duct 29 has a L-shape. In other words, the supply duct 29 is subdivided into a main portion (having the fluid inlet FI and extending along axis Y) and an end portion (having the fluid outlet FO) extending along an axis Z, substantially parallel to axis X. Axis Z may substantially coincide with axis X. The end portion is preferably pointing down towards the lower portion 27; i.e. the end portion is arranged such that the fluid outlet FO faces a distribution device 30 (as better described below).

The catalytic reactor 24 comprises the distribution device 30 which is configured to distribute the exhausted hydrogen peroxide solution entering the tubular housing 25.

The distribution device 30 is arranged within the tubular housing 25 below the supply duct 29; in other words, the distribution device 30 is arranged facing the supply duct 29, in particular facing the fluid outlet FO. Preferably, the distribution device 30 is arranged within the lower portion 27; in particular, the distribution device 30 is arranged in an upper part of the lower portion 27 in the area where the upper portion 26 and the lower portion 27 are connected.

The distribution device 30 comprises a distribution plate 31 provided with a number of through holes 32 configured for the passage of the exhausted hydrogen peroxide solution coming from the supply duct 29. The distribution plate 31 is preferably realized in a metal material, in particular stainless steel. The distribution plate 31 completely engages the lower portion 27; in other words, the distribution plate 31 has a circular shape with a diameter that approximates the inner diameter of the lower portion 27.

Advantageously, the distribution plate 31 comprises a plurality of through holes 32. Even more advantageously, the number of through holes 32 is comprised within eight and fifteen. The number of through holes 32 is determined such that the exhausted hydrogen peroxide solution drip onto the lower portion 27 at a desired rate.

Each through hole 32 is defined by a cylindrical lateral surface with a symmetry axis, preferably parallel to the axis X. The through holes 32 are preferably uniformly distributed on the available surface of the distribution plate 31. Advantageously, each through hole 32 has an inner diameter comprised between 0,1 and 0,2 cm; preferably, the inner diameter of each through hole 32 is 0,15 cm. Advantageously, the distribution plate 31 has a thickness along the longitudinal direction (i.e. along the axis X) comprised between 1,5 and 2,5 cm; preferably, the thickness of the distribution plate is 1,9 cm.

The catalytic reactor 24 further comprises a gas rising device 33 which is arranged within the tubular housing 25; in particular, the gas rising device 33 is arranged below the supply duct 29 and above the distribution plate 31.

The gas rising device 33 comprises a number of pipes 34 configured for the passage of vapor as better described in the following. Advantageously, the gas rising device 33 comprises a plurality of pipes 34. Even more advantageously, the number of pipes 34 is comprised within two and six. Preferably, the gas rising device 33 comprises three pipes 34.

Each pipe 34 is defined by a cylindrical lateral wall with a symmetry axis, preferably parallel to the axis X. Advantageously, each pipe 34 has an inner diameter comprised between 0,8 and 1 cm; preferably, the inner diameter of each pipe 34 is 0,9 cm. Each pipe 34 has an outer diameter comprised between 0,9 and 1,2 cm; preferably, the outer diameter of each pipe 34 is 1 cm.

Advantageously, each pipe 34 has a height along the longitudinal direction (along the symmetry axis) comprised between 1,5 and 2,5 cm; preferably, the height of each pipe 34 is 1,9 cm. Preferably, each pipe 34 comprises a respective superior covering element. Each pipe 34 is configured to convey the gas and aerosol flow coming from the lower portion 27 towards the upper portion 26. Each pipe 34 has an inlet for the gas and aerosol flow defined in the distribution plate 31. The covering element are instead configured to spread radially the gas and aerosol flow coming from the lower portion 27 towards the lateral wall of the upper portion 26.

In the upper portion 26 above the supply duct 29, it is defined an upper chamber UC wherein the catalyst reaction takes place. The upper chamber UC is delimited by a lateral and an upper wall of the upper portion 26 and by a support plate 35 arranged above the supply duct 29. More in detail, the support plate 35 comprises a steel mesh.

Similarly, in the lower portion 27 below the distribution plate 31, it is defined a lower chamber LC wherein the catalyst reaction takes place. The lower chamber LC is delimited by a lateral wall of the lower portion 27, by the distribution plate 31 and by a support plate 36 arranged at an exit end of the lower portion 27. More in detail, the support plate 36 comprises a steel mesh.

According to some embodiments, the apparatus may further comprise a third support plate, in particular being a steel mesh positioned under the distribution device 30. Such third support plate, if provided, is removable, so as to allow filling of lower portion 27.

Both the upper chamber UC and the lower chamber LC are at least partially filled with a catalyst bed. The catalyst bed is configured to accelerate a reaction of reduction of the exhausted hydrogen peroxide solution.

According to some embodiments, the catalyst bed (in particular, the first catalyst bed and the second catalyst bed) may include alumina particles (Al2O3) coated with metals (i.e. copper and manganese, or platinum, or palladium). The type and nature of the metal, if it is in the metallic or in an oxide form during operation should not limit this disclosure. In particular, the alumina particles coated with metals may be packed in the catalytic reactor 24 according to a packed bed structure. Alternatively, the alumina particles coated with metals may be provided in a monolith structure. A particularly effective solution has been found in using, as catalyzer, alumina particles coated with copper and manganese and provided in a packed bed structure.

According to some alternative embodiments, the catalyst bed (in particular, the first catalyst bed and the second catalyst bed) may include activated carbons. The activated carbons are a cheaper option, compared to alumina particles coated with metals.

The reaction of catalytic reduction of the exhausted hydrogen peroxide is exothermic; in other words, the reaction of catalytic reduction of the exhausted hydrogen peroxide generates an amount of heat. The reaction of catalytic reduction of the exhausted hydrogen peroxide solution provides as a result oxygen and water and, more in detail the water is provided both in the liquid phase and in the vapor phase. The lower catalyst bed is arranged within the lower portion 27 and is configured for the conversion of the liquid phase of the exhausted hydrogen peroxide solution entering the lower chamber LC through the distribution plate 31. The upper catalyst bed is instead arranged within the upper portion 26. The upper catalyst bed is configured for the conversion of the aerosol and gas phase coming from the lower chamber LC and entering the upper chamber UC through the pipes 34.

As already mentioned, the housing 25 extends along a vertical direction parallel to the weight force, and the lower catalyst bed is positioned below the upper catalyst bed with respect to the vertical direction. The lower catalyst bed is configured to receive the exhausted hydrogen peroxide solution in liquid phase entering the catalytic reactor 24 from the supply duct 29, and the upper catalyst bed is configured to receive the gaseous phase of the exhausted hydrogen peroxide solution coming from the lower catalyst bed, through the pipes 34.

The exhausted hydrogen peroxide solution enters the catalytic reactor 24 from the supply duct 29 in liquid phase and drip onto the lower catalyst bed through the distribution device 30. A portion of the exhausted hydrogen peroxide solution has an exothermic reaction of catalytic reduction, and the remaining portion of the exhausted hydrogen peroxide solution evaporates (due to heat generated by the exothermic reaction of catalytic reduction) and reaches the upper catalyst bed where a further exothermic reaction of catalytic reduction takes place.

The upper and lower catalyst bed are supported respectively by the support plates 35 and 36.

The water in liquid phase is drained out of the catalytic reactor 24 through a first pipe 37 which is in fluid communication with a lower end of the lower portion 27. The flow rate of the water in liquid phase exiting the catalytic reactor 24 through the first pipe 37 is comprised between 0.01 and 0.05 l/min; preferably, the flow rate of water in liquid phase exiting the catalytic reactor 24 through the first pipe 37 is 0.02 l/min.

The temperature of the water in liquid phase exiting the catalytic reactor 24 through the first pipe 37 is preferably, comprised between 75°C and 90°C; preferably, the temperature of the water in liquid phase exiting the catalytic reactor 24 through the pipe 37 is 90°C. However, in case a separator is provided, the temperature of the water in liquid phase exiting the catalytic reactor 24 through the first pipe 37 may be comprised between 25°C and 90°C, preferably around 35°.

A gaseous flow of oxygen and water is drained out of the catalytic reactor 24 through a second pipe 38 which is in fluid communication with an upper end of the upper portion 26. The flow rate of oxygen exiting the catalytic reactor 24 through the second pipe 38 is comprised between 1 and 2 Nm³/h (Normal cubic meter per hour); preferably, the flow rate of oxygen exiting the catalytic reactor 24 through the second pipe 38 is 1.5 Nm³/h. The flow rate of water in vapor phase exiting the catalytic reactor 24 through the second pipe 38 is comprised between 7 and 7.5 Nm³/h; preferably, the flow rate of water in vapor phase exiting the catalytic reactor 24 through the pipe 38 is 7.3 Nm³/h.

The temperature of the gaseous flow of oxygen and water exiting the catalytic reactor 24 through the pipe 38 is around boiling point (100°C). The gaseous flow of oxygen and water exiting the catalytic reactor 24 through the second pipe 38 comprises 83% mass of water in vapor phase and 17% mass of oxygen.

It is noted that the housing 25 encloses an inner environment, communicating with an outer environment (only) through the supply duct 29, the first pipe (7 and the second pipe 38.

The apparatus 17 for disposing of the exhausted hydrogen peroxide solution further comprises a separator 39 for the water in liquid phase. The separator 39 is arranged along the first pipe 37. The separator 39 is configured to separate the water in liquid phase and allow it to pass therethrough to be drained out. According to a preferred embodiment the separator 39 comprises a floating element; when the level of water in liquid phase rises within the separator 39, the floating element rises accordingly thereby opening an orifice for discharging exclusively the water in liquid phase. According to a further embodiment the separator 39 comprises a solenoid valve. The present description describes a method for disposing of the exhausted hydrogen peroxide solution used for the sterilization of the web 3 of packaging material. More in detail said method comprises the step of supplying, through the supply duct 29, the exhausted hydrogen peroxide solution into the catalytic reactor 24. The method further comprises a step of catalytic reduction of the exhausted hydrogen peroxide solution taking place within the catalytic reactor 24. The catalytic reduction of the exhausted hydrogen peroxide solution produces both water in liquid phase and a gaseous flow of oxygen and water. Finally, said method comprises the step of draining out of the catalytic reactor 24 the water in liquid phase, through the first pipe 37 and the gaseous flow of oxygen and water through the second pipe 38. Advantageously, the method may further comprise the step of cooling gaseous flow of oxygen and water exiting the catalytic reactor 24 through the second pipe 34.

The present description further describes a method for producing sealed packages containing a pourable product, from the web 3 of packaging material with the method step of sterilizing the web 3 of packaging material by advancing the web 3 of packaging material through a bath containing a hydrogen peroxide solution; and disposing the hydrogen peroxide solution, once exhausted, according to the method described above.

The apparatuses 17 for disposing of the exhausted hydrogen peroxide solution described above has some advantages; in particular, it provides for good results in a short time process with no consumption of water.

## Claims

1. An apparatus (17) for disposing of an exhausted hydrogen peroxide solution used for the sterilization of a web (3) of packaging material, the apparatus (17) comprising:
- a catalytic reactor (24) configured for the catalytic decomposition of the exhausted hydrogen peroxide solution, wherein the catalytic reactor (24) comprises a housing (25) containing at least one catalyst bed,
the apparatus (17) further comprising:
- a disposal tank (21) configured for containing the exhausted hydrogen peroxide solution;
- at least a supply duct (29) configured for supplying the exhausted hydrogen peroxide solution from the disposal tank (21) into the housing (25);
- a first pipe (37) connected to the housing (25);
- a second pipe (38) connected to the housing (25);
wherein the catalytic reactor (24) is configured to produce water in liquid phase to be drained out through the first pipe (37) and a gaseous flow of oxygen and water to be drained out through the second pipe (38).

2. The apparatus (17) according to claim 1, wherein the housing (25) encloses an inner environment, communicating with an outer environment through the supply duct (29), the first pipe (37) and the second pipe (38).

3. The apparatus (17) according to claim 2, wherein the housing (25) extends along a vertical direction parallel to the weight force, and the catalytic reactor (24) comprises a first catalyst bed and a second catalyst bed being positioned below the first catalyst bed with respect to the vertical direction; wherein the second catalyst bed is configured to receive the exhausted hydrogen peroxide solution in liquid phase from the supply duct (29) and the first catalyst bed is configured to receive a gaseous phase of the exhausted hydrogen peroxide solution coming from the first catalyst bed.

4. The apparatus (17) according to claim 3, and comprising:
- a first support plate (35) arranged within the housing (25) for supporting the first catalyst bed;
- a second support plate (36), being arranged within the housing (25) for supporting both the second catalyst bed;
wherein the supply duct (29) is configured to supply the exhausted hydrogen peroxide solution in between the first support plate (35) and the second support plate (36).

5. The apparatus (17) according to claim 4, further comprising a distribution device (30) which is arranged within the housing (25) below the supply duct (29) and above the second support plate (36) and is configured to distribute the exhausted hydrogen peroxide solution in liquid phase to the first catalyst bed, wherein the distribution device (30) comprises a distribution plate (31) provided with a number of through holes (32) configured for the passage of the exhausted hydrogen peroxide solution

6. The apparatus (17) according to any claims 4, wherein the supply duct (29) is provided with a fluid outlet (FO) which is configured to direct the exhausted hydrogen peroxide solution onto the distribution device (30); wherein the fluid outlet (FO) faces the distribution device (30).

7. The apparatus (17) according to any of claims from 3 to 6, wherein the catalytic reactor (24) further comprises a gas rising device (33) which is arranged within the housing (25), in particular below the supply duct (29), and comprises a number of pipes (34) configured for conveying the gaseous phase of the exhausted hydrogen peroxide solution coming from the second catalyst bed towards the first catalyst bed.

8. The apparatus (17) according to any one of the previous claims, and further comprising a separator (39) arranged along the first pipe (37) and configured to separate the water in liquid phase and allow it to pass therethrough to be drained out.

9. The apparatus (17) according to any one of the previous claims, further comprising feeding means (22), which are configured to draw the exhausted hydrogen peroxide solution out the disposal tank (21) and to supply said solution to the catalytic reactor (24) through the supply duct (29).

10. The apparatus (17) according to claim 9, wherein the feeding means (22) are configured to draw the exhausted hydrogen peroxide solution, being in the liquid phase, out the disposal tank (21) and to supply the hydrogen peroxide solution, still being in the liquid phase, to the at least one catalyst bed of the catalytic reactor (24).

11. The apparatus (17) according to any one of the previous claims, further comprising a chemical filter positioned upstream of supplying duct (29) and configured to remove from the hydrogen peroxide solution a stabilizer content, before the hydrogen peroxide solution is supplied into the housing (25).

12. A packaging machine comprising:
- a conveying apparatus (9) for advancing a web (3) of packaging material along a conveying path (P);
- a sterilization unit (15) to sterilize the advancing web (3) of packaging material having a sterilizing bath, in which a chemical sterilizing agent comprising a hydrogen peroxide solution is applied to the web (3) of packaging material;
- a tube forming and sealing device (9) arranged downstream of the sterilization unit (15) along the conveying path (P) and configured to gradually fold the web (3) of packaging material into a tube (4) and longitudinally sealing the tube (4);
- a filling device (10) for filling the tube (4) with the pourable product; and
- an apparatus (17) for disposing of the exhausted hydrogen peroxide solution used in the sterilization unit (15) realized according to any one of the previous claims, wherein the disposal tank (21) of the apparatus (17) is configured to collect the hydrogen peroxide solution from the sterilizing bath of the sterilization unit (15) of the packaging machine.

13. A method for disposing of an exhausted hydrogen peroxide solution used for the sterilization of a web (3) of packaging material, the method comprising:
- supplying the exhausted hydrogen peroxide solution, being in liquid phase, into at least one catalyst bed of a catalytic reactor (24), through a supply duct (29);
- producing water in liquid phase and a gaseous flow of oxygen and water within the catalytic reactor (24);
- draining out of the catalytic reactor (24) the water in liquid phase, through a first pipe (37), and the gaseous flow of oxygen and water, through a second pipe (38).

14. The method according to claim 13, wherein the step of supplying the exhausted hydrogen peroxide solution includes: drawing the exhausted hydrogen peroxide solution, being in the liquid phase, out of a disposal tank (21), and supplying the hydrogen peroxide solution, still being in the liquid phase, to the at least one catalyst bed of the catalytic reactor (24), through the supply duct (29).

15. A method for producing sealed packages containing a pourable product from a web (3) of packaging material, the method comprising:
- sterilizing the web (3) of packaging material by advancing the web (3) through a bath containing a hydrogen peroxide solution;
- disposing the hydrogen peroxide solution, once exhausted, according to the method of claim 13 or 14,
- folding the web (4) into a tube (4);
- longitudinally sealing the tube (4);
- filling the tube (4) with the pourable product;
- forming and transversally sealing the tube (4) at successive cross-sections thereof, thereby obtaining a plurality of sealed packages.
